# EUROPEAN PATENT SPECIFICATION

(11) **EP 4 290 264 B1**
(45) Date of publication and mention of the grant of the patent: **15.01.2025**
(21) Application number: 23176705.4
(22) Date of filing: 01.06.2023
(51) Int. Cl.: G01R 33/56, G01R 33/54, G16H 30/20, G16H 30/40, G16H 50/20

(54) **MEDICAL IMAGE PROCESSING APPARATUS, DIAGNOSTIC IMAGING APPARATUS, AND MEDICAL IMAGE PROCESSING METHOD**
VORRICHTUNG ZUR VERARBEITUNG MEDIZINISCHER BILDER, VORRICHTUNG ZUR DIAGNOSTISCHEN BILDGEBUNG UND VERFAHREN ZUR VERARBEITUNG MEDIZINISCHER BILDER
APPAREIL DE TRAITEMENT D'IMAGE MÉDICALE, APPAREIL D'IMAGERIE DE DIAGNOSTIC ET PROCÉDÉ DE TRAITEMENT D'IMAGE MÉDICALE

(30) Priority: 09.06.2022 JP 2022093909
(43) Date of publication of application: 13.12.2023
(73) Proprietor: FUJIFILM Corporation, Tokyo 106-8620 (JP)
(72) Inventor: YOKOSAWA, Suguru, Kashiwa-shi, Chiba, 277-0804 (JP); SUZUKI, Atsuro, Kashiwa-shi, Chiba, 277-0804 (JP); SHIRAI, Toru, Kashiwa-shi, Chiba, 277-0804 (JP)
(74) Representative: Strehl Schübel-Hopf & Partner

(56) References cited:
- EP-A2- 3 187 891
- US-A1- 2011 110 572
- US-A1- 2021 042 913
- US-B2- 9 805 470

## Description

### BACKGROUND OF THE INVENTION

### Technical Field

The present invention relates to processing of a medical image acquired by an apparatus such as an MRI apparatus and a CT apparatus. More particularly, the present invention relates to an image processing technique including an image quality adjusting function.

### Description of the Related Art

A diagnostic image obtained by imaging with an MRI apparatus is generally not a quantitative image in which pixel values are associated with physical quantities, but an image enhancing physical quantitative values such as longitudinal relaxation time (T1) and transverse relaxation time (T2) of a tissue, and thus the image is not standardized. Further, a quality of an outputted image depends on imaging conditions such as imaging parameters and post-processing conditions such as filtering (image reconstruction conditions), and there are enormous number of output patterns of the image quality, even in the same MRI apparatus. Further, since the image quality also depends on vendors and device specifications, the image quality may be different depending on a device model, even under the same imaging conditions. Therefore, there is a need for adjusting the image quality to satisfy a desire of a user, e.g., a doctor, who performs a diagnosis using the MRI apparatus. In order to adjust the image quality as desired by the doctor, however, there are required trial and error to perform imaging repeatedly while varying adjustment parameters that affect the image quality, and thus it is not easy to adjust the image quality.

In connection with the problem as described above, JP-A-2021-27891 (hereinafter, referred to as Patent Literature 1) discloses a technique of applying a trained model to medical image data so as to generate imaging parameters for the medical image data. In this method, when a user selects a medical image of a desired mode, imaging parameters associated with the selected medical image are outputted, and imaging is performed using the imaging parameters. Examples of the imaging parameters include types of collecting system (coil, etc.), types of collecting method (pulse sequence, etc.), types of temporal parameters (TE, TR, etc.), flip angle, imaging cross-section, types of reconstruction, FOV, matrix size, slice thickness, the number of phase encoding steps, scanning option, and so on.

Another example of a conventional imaging system is described in US 2011/0110572 A1. In this example, a set of gold standard images is used for dynamically improving the quality of medical imaging.

### SUMMARY OF THE INVENTION

### Technical Problem

In the technique as described in Patent Literature 1, the user designates an image whose imaging parameters are unknown with respect to a mode of a medical image, for example, a T1 enhanced image or a T2* enhanced image, or a DWI (diffusion weighted image), or further a map image. Then, the imaging parameters associated with the mode of the designated medical image are estimated. This technique, however, does not aim to perform a desired image quality adjustment nor to present parameters for obtaining a desired image quality.

When the technique of Patent Literature 1 is tried to be applied for the purpose of image quality adjustment, medical images matching a desired image quality are required in developing the trained model. But what image quality is favorable is determined from a personal point of view of a user, e.g., a doctor, and a desired medical image is not necessarily included in the already-existing image data. Further, as described above, the image quality also depends on the vendors and the device specifications. Accordingly, even if a desired image quality is inputted and imaging conditions are obtained for the medical data matching the desired image quality, the obtained image quality may be different even under the same imaging conditions, when the device model is different. Thus, even when the technique described in Patent Literature 1 is employed, it is not easy to perform adjustment so as to achieve a desired image quality in a particular apparatus to be applied.

An object of the present invention is to provide a means that facilitates presentation of conditions to obtain the image quality desired by the user, and this achieves savings in time and effort required for repetitive image quality adjustment.

### Solution to Problem

In order to solve the above problem, the present invention provides the medical image processing apparatus defined in claim 1. It uses a database associating an image feature value extracted from image quality data, with adjustment parameters that affect an image quality, and on the basis of the image quality data inputted by the user, the present invention presents the adjustment parameters allowing obtainment of the image quality that is the same as or close to the image quality data inputted by the user. The adjustment parameters include various parameters such as imaging conditions and image reconstruction conditions.

That is, a medical image processing apparatus of the present invention comprises a reception unit configured to receive image quality data reflecting a user's desire for an image quality of a medical image, a feature value calculation unit configured to calculate an image feature value with respect to the image quality data, and a parameter presentation unit configured to use the database associating adjustment parameters related to the image quality with the image feature value, to determine and present the adjustment parameters suitable for the image quality data received by the reception unit.

In addition, a diagnostic imaging apparatus of the present invention includes an image processing unit comprising the above-described configuration of the medical image processing apparatus.

Further, a medical image processing method of the present invention as defined in appended claim 13, comprises receiving image quality data reflecting a user's desire for an image quality of a medical image, calculating an image feature value with respect to the image quality data, and using a database associating adjustment parameters related to the image quality with the image feature value, to determine and present the adjustment parameters suitable for the image quality data being received.

According to the present invention, by simply inputting the image quality data, it is possible to present recommended imaging parameters that allow acquisition of an image with the image quality close to the user-desired image quality, thereby adjusting the image quality to the desired quality in a short time.

### BRIEF DESCRIPTION OF THE DRAWINGS

FIG. 1 illustrates an overall configuration of a diagnostic imaging apparatus;
FIG. 2 illustrates a processing of an image processing apparatus;
FIG. 3A is a functional block diagram showing an example of the image processing apparatus according to a first embodiment;
FIG. 3B is a functional block diagram showing another example of the image processing apparatus according to the first embodiment;
FIG. 4 illustrates an example of a process for receiving image quality data according to the first embodiment;
FIG. 5 illustrates a structure of a database according to the first embodiment;
FIG. 6 illustrates a data extraction process according to the first embodiment;
FIG. 7 illustrates one example of the data extraction process according to the first embodiment;
FIG. 8 illustrates an example of the database structure and extracted data according to a modification of the first embodiment;
FIG. 9 illustrates a display example of the first embodiment;
FIG. 10 is a functional block diagram showing the image processing apparatus according to a second embodiment;
FIG. 11 illustrates processing steps of the second embodiment; and
FIG. 12 illustrates processing steps of a modification of the second embodiment.

### DETAILED DESCRIPTION OF THE PREFERRED EMBODIMENTS

There will now be described embodiments of a medical image processing apparatus of the present invention with reference to the accompanying drawings. The medical image processing apparatus of the present invention may serve as a function of an operation part incorporated in a diagnostic imaging apparatus such as an MRI apparatus, a CT apparatus, and an ultrasound imaging apparatus, or may be an image processing apparatus independent of those kinds of diagnostic imaging apparatuses.

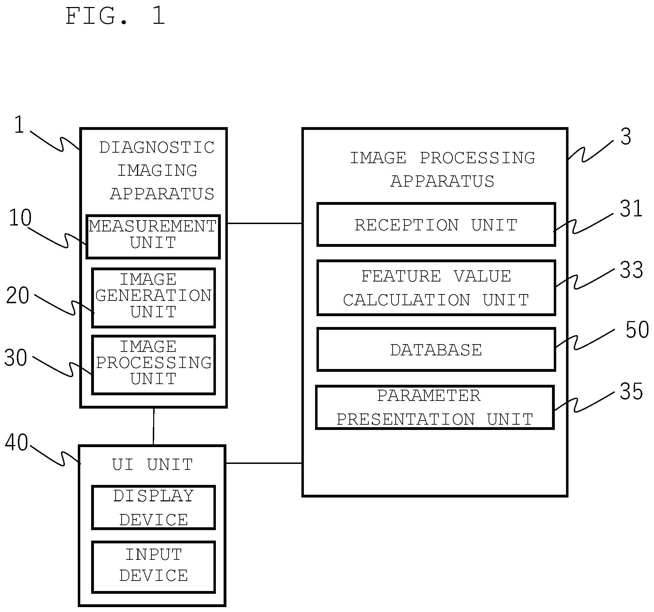

As shown in FIG. 1, when the image processing apparatus serves as one function of the diagnostic imaging apparatus, the diagnostic imaging apparatus 1 includes a measurement unit 10 configured to collect measurement data of a subject, an image generation unit 20 configured to generate an image using the measurement data acquired by the measurement unit 10, and an image processing unit 30 configured to process the image generated by the image generation unit 20.

When the medical image processing apparatus (hereinafter, simply referred to as an image processing apparatus) 3 independent of the diagnostic imaging apparatus 1 is provided, the image processing apparatus 3 or the image processing unit 30 includes a reception unit 31 configured to receive specific image data (hereinafter, collectively referred to as image quality data) reflecting a desire of a doctor or a technician (hereinafter, collectively referred to as a user) regarding the image quality of a medical image, a feature value calculation unit 33 configured to calculate an image feature value that specifies the received image quality data, and a parameter presentation unit 35 configured to use a database associating adjustment parameters of from taking the image until generating the image, with the image feature value, to determine and present an optimum adjustment parameters of the image data having the desired image quality. Further, there may also be provided a database 50 containing a large number of data necessary for the processing that will be described later.

The diagnostic imaging apparatus 1 (the image processing unit 30) and the image processing apparatus 3 are provided with a UI unit 40 configured to receive an input from the user and to present a processing result to the user. The UI unit 40 comprises an input device and a display device (output device) for interactive exchange with the user.

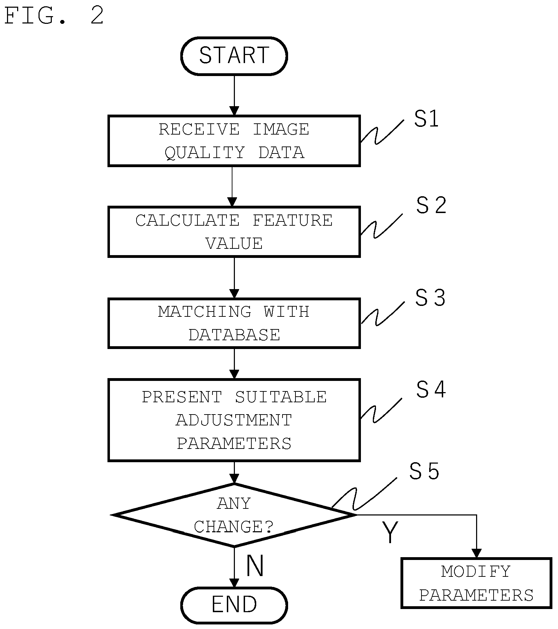

There will now be described an outline of the processing in the image processing unit 30 or in the image processing apparatus 3, using the image processing apparatus 3 as a representative. FIG. 2 illustrates the outline of the processing.

When the reception unit 31 receives information (image quality data) related to the image quality of the image inputted via the UI unit 40 (S1), the feature value calculation unit 33 uses thus inputted information to calculate the image feature value of the image quality desired by the user (S2). The information (image quality data) related to the image quality inputted by the user may be, for example, an image itself having the image quality ideal for the user, including image data and its accompanying information, or information received through a selection of a desired image from a plurality of candidate images presented by the image processing apparatus 3. Entry of such information determines specific image data, i.e., the image quality data reflecting the user's desire for the image quality, and then the feature value calculation unit 33 calculates the image feature value of the image quality data using a general analysis method.

Meanwhile, the database 50 is prepared in advance, with respect to each of various images, associating an image having its adjustment parameters upon acquiring and generating the image, with an image having its feature value. The adjustment parameters may include, for example, imaging conditions (such as various imaging parameters and device conditions), and conditions at the time of image generation (such as reconstruction conditions, and conditions for filtering and noise reduction processing). Further, the database 50 may be created for each modality, or the type of modality may be included in the adjustment parameters. The database 50 may be a part of the image processing apparatus 3, or it may be prepared separately from the image processing apparatus 3 and accessible therefrom.

The parameter presentation unit 35 compares the image feature value for the predetermined image quality data calculated by the feature value calculation unit 33, with the feature values of the images stored in the database 50, specifies the closest feature value, and presents the adjustment parameters (adjustment parameter set) associated with the feature value, to the user via the UI unit 40, as suitable adjustment parameters (S3). Further, the suitable adjustment parameters specified by the parameter presentation unit 35 may be transferred to the diagnostic imaging apparatus 1, and the measurement unit 10 and the image generation unit 20 of the diagnostic imaging apparatus 1 may collect the measurement data and generate the image in accordance with the suitable adjustment parameters (S4). At this time, it is further possible that the adjustment parameters are presented to the user, to be modified as appropriate, and then the modified parameters are passed to the measurement unit 10 and the image generation unit 20 (S5). In the case where the image processing unit 30 of the diagnostic imaging apparatus 1 specifies the suitable adjustment parameters, the above-described steps S4 and S5 are performed as the processing within the apparatus.

According to the present embodiment, the adjustment parameters desired by the user can be easily set and applied to the diagnostic imaging apparatus. This eliminates the need for trial and error in order for the user to obtain a desired image quality, enabling generation of an image of a desired quality in a short time.

In view of the above-described configuration of the image processing apparatus and the outline of the processing thereof, embodiments of the detailed processing will be described below.

### First Embodiment

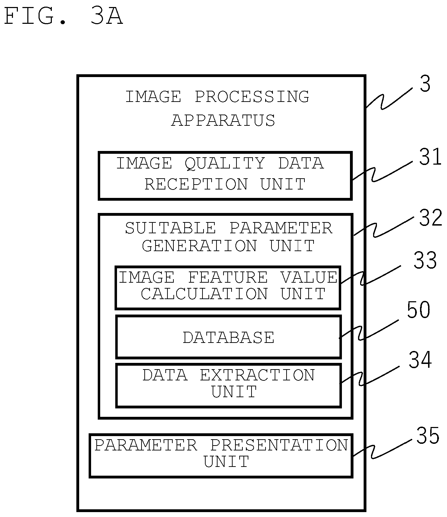

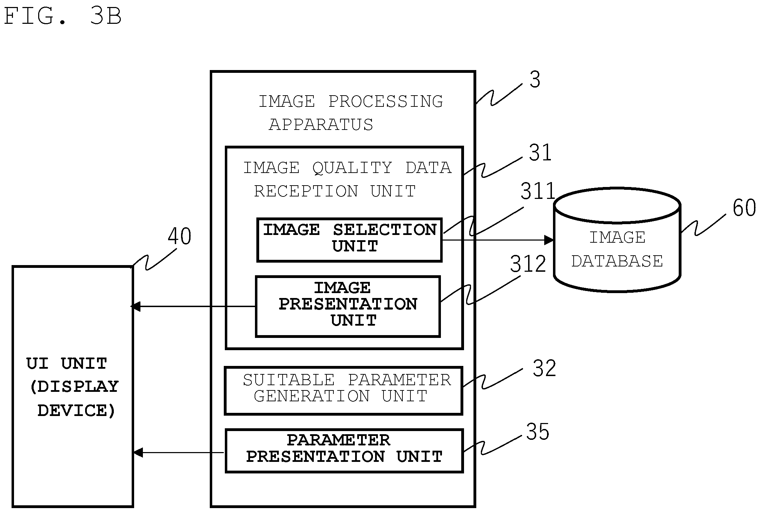

FIGs. 3A and 3B (referred to as FIG. 3 when referenced collectively) show the configuration of the image processing apparatus according to the present embodiment. In FIG. 3, the same elements as those shown in FIG. 1 are denoted by the same reference numerals. As illustrated, the image processing apparatus 3 of the present embodiment includes an image quality data reception unit (hereinafter, simply referred to as the reception unit) 31, a suitable parameter generation unit 32, and a parameter presentation unit 35. The suitable parameter generation unit 32 includes an image feature value calculation unit 33 (feature value calculation unit), a database 50, and a data extraction unit 34. The data extraction unit 34 performs matching between the feature value stored in the database 50 and the feature value calculated by the feature value calculation unit 33, and extracts data stored in the database.

The reception unit 31 has the same function as that of the reception unit 31 in FIG. 1, but as shown in FIG. 3B, it may also have functions (an image selection unit 311 and an image presentation unit 312) for selecting and presenting an image from an image database 60.

There will now be described the processing of the present embodiment. Although the image data as a target in the present embodiment is not limited to an MR image, there will be described a case where the image targeted in the present embodiment is an image acquired by MRI, as an example.

### [Receive image quality data: S1]

First, the reception unit 31 receives information related to an image quality inputted by the user. In the case where the user owns the image data of the user's desired image quality (which represents a situation not covered by the scope of the claims), this inputted information related to the image quality is data of DICOM (Digital Imaging and Communications in Medicine). The DICOM information includes image data, and as accompanying information, tag information such as a resolution, matrix size, and TE, TR, which are imaging parameters. By using the DICOM information, it is possible to restrict a selection range of the adjustment parameters in the subsequent steps, thereby facilitating data extraction from the database.

Here, the user can specify a data path of the DICOM information via the UI unit 40, thereby entering the image quality data, that is, establishing the interface between the reception unit 31 and the user. In this case, the image quality data received by the reception unit 31 is preferably a DICOM image, but when the user possesses only the image data of the desired image quality (without accompanying information), only the image data may be received.

The invention addresses the case where the user does not have any particular image of the ideal image quality, that is, the user has an ideal image quality only as a concept, not in the form of a specific image. For this reason, the image selection unit 311 (FIG. 3B) first displays in accordance with the invention a plurality of images as candidates on the display device, from the image database 60, and prompt the user to select one or more images. The image database 60 stores, for example, various images having different conditions on the image quality, such as SNR, contrast, and denoising strength, for each modality and each site, and for each image types (such as T1 enhanced images, T2 enhanced images, and DWI) if the images are MR images. Databases such as image databases already placed in the public domain (databases as public properties) and image databases held by facilities may be available as the image database 60. Alternatively, a database newly created may be used as the image database 60.

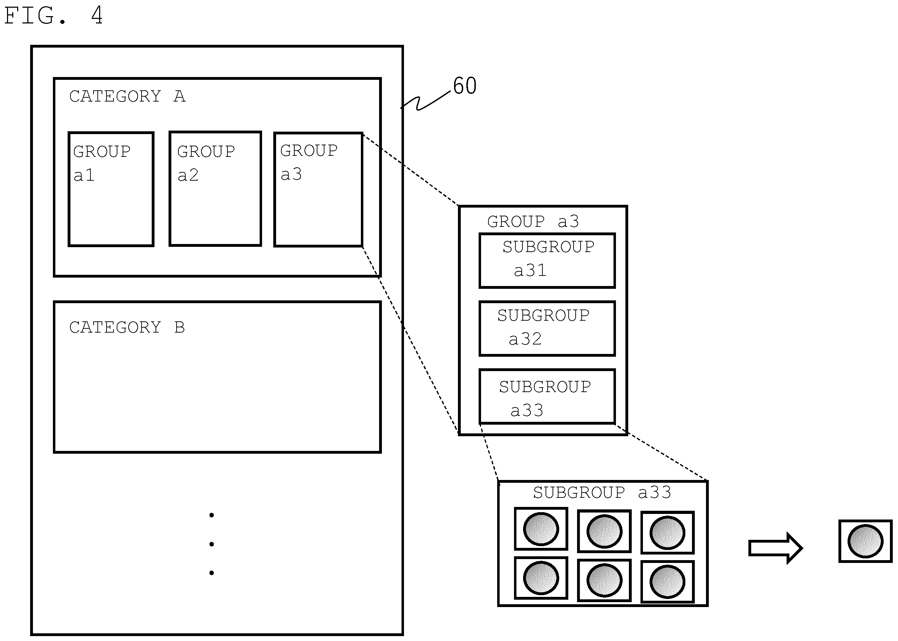

A method of the image selection unit 311 to select a plurality of candidate images from the database is not limited to a specific method, but it is preferable to receive a multi-stage selection to achieve efficient selection. As illustrated in FIG. 4, for example, the user is allowed to select a category to which the images belong, and then to select a plurality of candidate images included in one group among multiple images included in the category. Then, the image presentation unit 312 presents the selected candidate images. Subsequently, receiving the user's selection and presenting the images are repeated in multiple stages, that is, after receiving the user's selection of the presented candidate images, multiple images included in a subgroup, which is the subordinate group of the selected one group, are selected and presented. Segmentation into categories or groups depends on the structure of the image database, and it can be based on the DICOM information stored with the images in the image database. For example, categorization can be performed based on the modalities, sites, image types, objective indicators (such as a matrix size, SNR, and contrasts) that determine the quality of the image, and so on.

In the case where the image is an MR image, for example, a selection of the image type of the MR image and a targeted site is received first. When the image type and the site are determined, various matrix sizes of a representative image (a freely selected image) out of multiple (a group of) images are presented, from a plurality of images of the image type and of the site, then prompting the user to select the matrix size. It is also possible to present the matrix sizes themselves for the selection, but presenting the various-sized images of the representative image can facilitate the user's selection of the matrix size. Once the matrix size is received, an image having the selected matrix size is presented. Also in the case above, it is possible to sequentially narrow the selection range such that lower classification (subgroup) (e.g., pulse sequence type) is presented or representative images belonging to the subgroup are presented to make a further selection. In here, there has been taken as an example the case where the upper group is made based on the matrix size, and the subgroup is made based on the pulse sequence type. However, the grouping is not limited to this example, and any category can be defined as the upper group in advance.

In a final step, the image presentation unit 312 presents a number of images to the extent reducing the burden on the user, and the user selects an image in response to the presentation of the images. Then, the reception unit 31 receives the selected image as image quality data. The number of received images is not limited to one, and more than one image, for example, two to several images, may be received. By receiving multiple images, it is possible to present as recommended adjustment parameters in a subsequent process, adjustment parameters associated with an image which is much closer to the image quality desired by the user.

### [Calculate feature value: S2]

When the image quality data is specified in step S1, the feature value calculation unit 33 calculates a feature value of the specified image quality data. The types of the feature value include, for example, SNR (signal-to-noise ratio), PSNR (Peak Signal to Noise Ratio), SSIM (Structural Similarity), CNR (Contrast to Noise Ratio), MSE (Mean Squared Error), sharpness, and so on. As method of calculating these feature values, general methods are known and specific descriptions thereof will not be provided here. It is also possible to employ a feature value that can be derived by using CNN, instead of or in combination with the objective feature values for which the calculation methods are already established. The feature value calculation unit 33 calculates one or more feature values.

More than one feature value can be represented as a one-dimensional vector into which individual feature values are concatenated.

### [Determine and present suitable parameters: S3, S4]

The data extraction unit 34 compares the feature value calculated by the feature value calculation unit 33 with the feature values of a large number of images stored in the database 50, and extracts the adjustment parameters (suitable parameters) associated with the feature value (S3), so that the parameter presentation unit 35 presents suitable parameters.

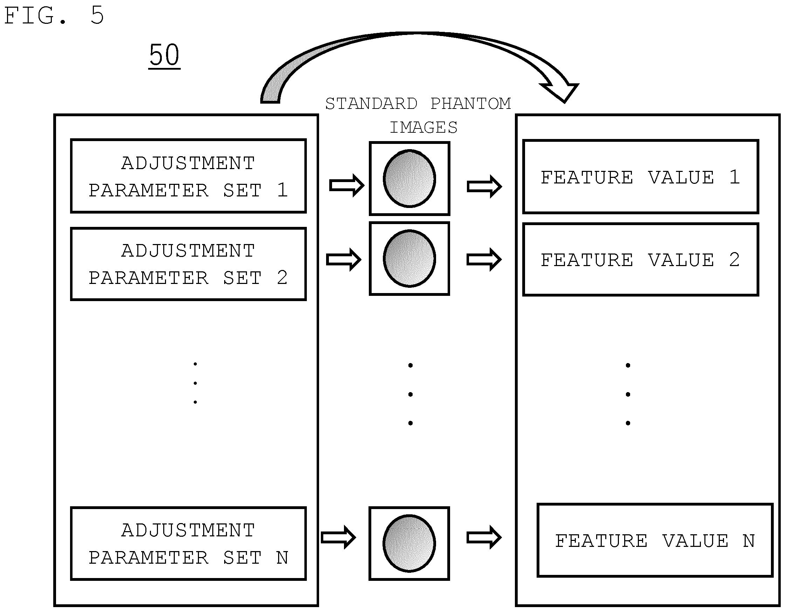

As shown in FIG. 5, for example, the database 50 can be created with images obtained by differentiating adjustment parameters variously, and feature values of the images, using standard phantoms obtained by standardizing respective sites. The database 50 holds the adjustment parameters (plural adjustment parameter sets) and the feature values of the images as discrete data. Instead of or in addition to the data using the standard phantoms, it is possible to link the adjustment parameters with the feature values of the images, with regard to a large number of images whose adjustment parameters are already known.

The database 50 may also retain, respectively for the adjustment parameter set and the feature values, interpolated data interpolating the discrete data and associating the adjustment parameters with the feature values. With this configuration, it is possible to hold the database that includes the feature value closer to the feature value calculated by the feature value calculation unit 33, allowing presentation of the adjustment parameters associated with the image closer to the image quality desired by the user, as the recommended adjustment parameters.

Types of the feature value in the database 50 do not necessarily have to coincide with the types of the feature value calculated by the feature value calculation unit 33. However, at least a part of the types overlaps with each other and they are calculated by the same method as the method that is used when the feature value calculation unit 33 calculates the feature value.

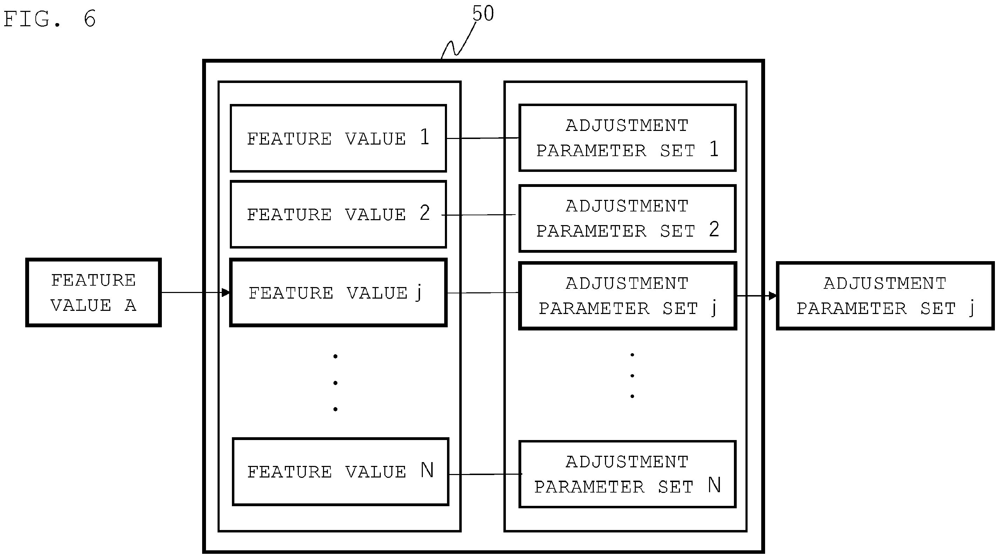

As shown in FIG. 6, the data extraction unit 34 performs matching between the feature the feature value calculated by the feature value calculation unit 33, that is, the feature value A of the image related to the image quality data received by the reception unit 31, and the feature values 1 to N stored in the database 50. Then, the closest feature value (the feature value j in FIG. 6) is extracted from the feature values in the database 50. The similarity between the comparing feature values is determined by the calculation such as subtraction, a correlation, or an inner product, between the feature values being one-dimensional vectors, and then, the feature value having the minimum difference, the highest correlation, or the largest inner product is determined and selected as the closest feature value. When there is more than one feature value at the closest position, such multiple feature values may be selected.

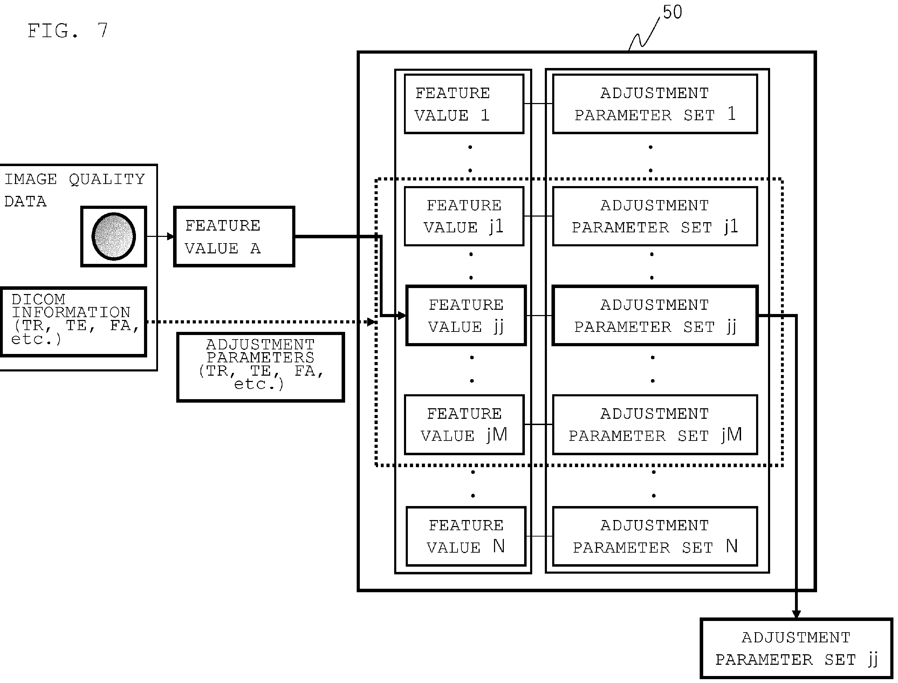

The parameter presentation unit 35 selects adjustment parameters (a combination of plural adjustment parameters: adjustment parameter set) associated with the feature value selected by the data extraction unit 34. As shown in FIG. 7, when the image quality data received by the reception unit 31 includes the DICOM information (tag information such as resolution, matrix size, and TE and TR), multiple data items having the adjustment parameters close to the adjustment parameters included in the DICOM information are extracted, the feature values in the multiple extracted data items are compared to select the adjustment parameters. This facilitates selection of the adjustment parameters from the database.

Alternatively, the adjustment parameters included in the DICOM information may be added as the feature values of the images, to perform matching with the feature values stored in the data base 50 and the feature values 1 to N calculated by the feature value calculation unit 33. In any of the above cases, the adjustment parameters included in the DICOM information and the adjustment parameters associated with the image close to the image quality desired by the user can be presented as the recommended adjustment parameters.

The parameter presentation unit 35 presents the selected adjustment parameters as the recommended adjustment parameters. At this time, it is also possible to add a function that allows the user to edit the presented recommended adjustment parameters.

The user performs imaging with the diagnostic imaging apparatus 1 using the recommended adjustment parameters presented by the parameter presentation unit 35, or the recommended adjustment parameters selected or modified by the user. When the image processing unit 30 of the diagnostic imaging apparatus has a function of presenting recommended adjustment parameters, measurement using the presented or appropriately modified recommended adjustment parameters is performed and then an image is generated after the post-processing conditions are applied.

According to the present embodiment, it is possible to easily set conditions for obtaining an image of the image quality desired by the user in the diagnostic imaging apparatus (imaging apparatus). Further, even when the user does not have information about a specific image quality, various images are presented for the user's selection, thereby allowing the user to clarify a desired image quality and performing the condition setting for obtaining the image quality.

### First Modification

In the above-described embodiment, multiple feature values are treated equally to specify the image quality. When multiple feature values are used, weights may be assigned to the feature values. For example, in the case where multiple feature values are added up and represented as a one-dimensional vector, a weight may be assigned to the feature value to reflect the user's preference followed by adding up the feature values, instead of simple addition.

As a method of reflecting the user's preference in the weights, for example, the user may select what is to be emphasized among the feature values such as the above-described SNR and CNR, and the weight of the particular feature value may be increased at a predetermined ratio (such as 1.5 times and 2 times of the weight of other feature values). As another method, the operation for the user to select an image is repeated along with shuffling the selection-target images, thereby obtaining the weight indicating what the user emphasizes.

### Second Modification

The present modification is the same as the first embodiment in the point that the image quality data is received and then matching is performed between the image feature value of the received data and the image feature values stored in the database. In the present modification, the database 50 stores not only the feature values and the adjustment parameters but also the images associated therewith, and when a set of suitable adjustment parameters is selected by the parameter presentation unit 35, an image associated therewith is presented together.

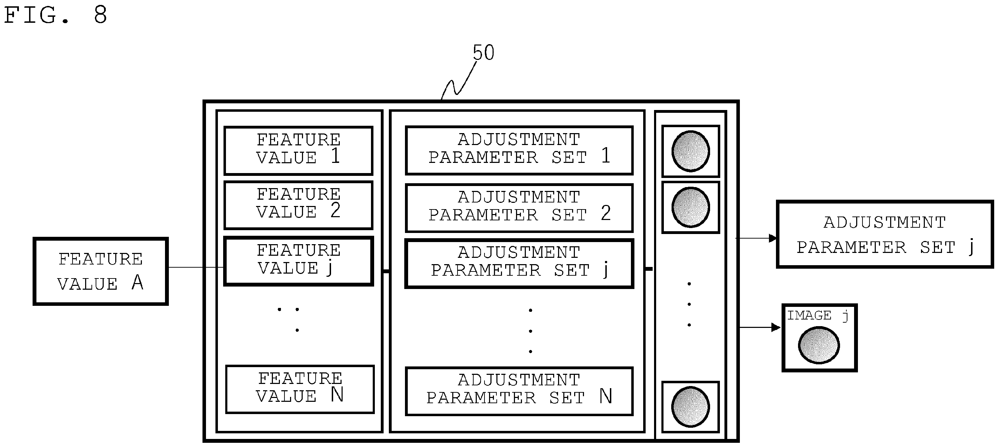

FIG. 8 illustrates an example of the database 50 according to the present modification.

In the case where the database is made up of various images obtained by differentiating the adjustment parameters and the feature values of the images, with the use of standard phantoms standardizing each site, the images (virtual images) used for creating the database can be used without any change, as the images associated with the adjustment parameters.

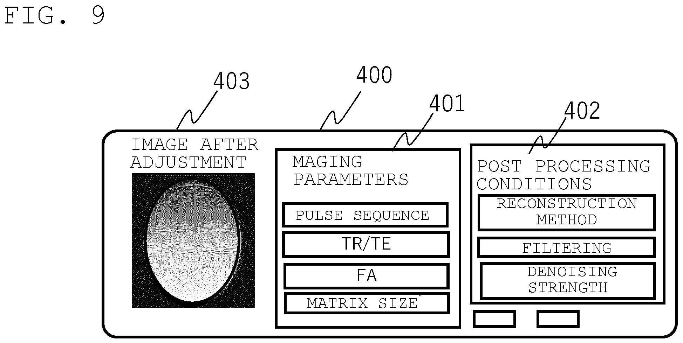

As illustrated in FIG. 9, for example, there is displayed on the display screen 400 of the UI unit 40 (display device), the image 403 associated with the selected suitable adjustment parameters (recommended adjustment parameters) 401 and 402. In this example, the adjustment parameters include the imaging parameter set 401 and the post-processing conditions 402 such as the image reconstruction conditions, and both are displayed. The user checks the image (virtual image) displayed on the display device, and confirms whether the adjustment parameters indicate the conditions for obtaining the image of the desired image quality. In the case where two or more adjustment parameter sets are selected and two or more images are displayed, a GUI may be displayed prompting the user to select an image that the user considers to be the more preferable, and this enables receiving of the user's selection.

According to the present modification, the user can check the displayed virtual image, to confirm the image quality under the condition that the recommended adjustment parameters are used, allowing the user to obtain the adjustment parameters more suitable for the desired image quality.

### Second Embodiment

In the present embodiment, the reception unit 31 of the image processing apparatus 3 receives the image quality data and presents the adjustment parameters in the same manner as in the first embodiment. The present embodiment features that a constraint condition for the adjustment parameters is received to present optimum adjustment parameters from among the adjustment parameters meeting the constraint condition.

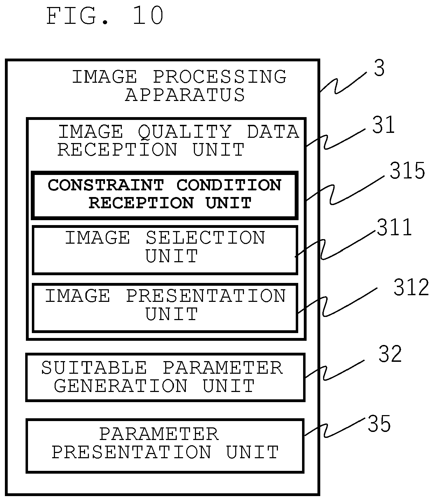

As shown in FIG. 10, the image processing apparatus 3 of the present embodiment comprises a constraint condition reception unit 315 to receive the constraint condition from the user as one function of the reception unit 31, in addition to the configuration of the first embodiment as shown in FIGs. 3A and 3B. When the reception unit 31 includes the image selection unit 311, the image selection unit 311 is provided with a function of selecting only the images acquired with the adjustment parameters that meet the constraint condition when the images included in the image database 60 are segmented.

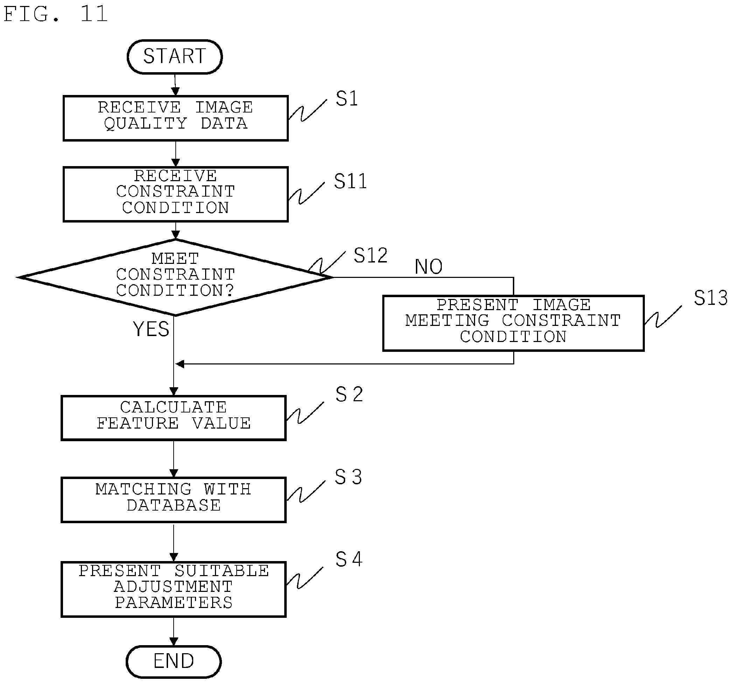

With reference to FIG. 11, there will now be described the processing of the image processing apparatus 3 according to the present embodiment, focusing on the points different from those of the first embodiment. In FIG. 11, the same processing steps as those shown in FIG. 2 are denoted by the same reference numerals, and different points will be mainly described.

First, the reception unit 31 receives constraints on the adjustment parameters together with the image quality data (S11). The constraints may include any of the restrictions on the imaging parameters and the restrictions on the post processing, but there may be constraints automatically determined from the specifications of a usable imaging apparatus, or constraints determined by the user's request. The latter constraints may include, for example, the imaging time, FOV, the number of slices (slice thickness).

To receive the constraint condition, it is possible to display a GUI listing items of the constraint conditions via the UI unit 40, allowing the user to enter a desired value, a degree, and so on. Entering the image quality data is the same as in the first embodiment, and when there is image data of a desired image quality, the image data may be inputted by specifying the data path. On the other hand, when there is no desired image data, a plurality of candidate images may be presented from the images in the database, in stages as appropriate according to the number of images.

Upon receiving the image quality data (S1), if the received image quality data includes the DICOM information, the reception unit 31 determines whether or not the information included in the DICOM information meets the constraints (S12). If it is determined that the information meets the constraints as a result of the determination, the feature value calculation unit 33 calculates the image feature value in the same manner as in the first embodiment. Then, as shown in FIG. 7, the data extraction unit 34 uses the DICOM information and the image feature value (feature value A) to select the suitable adjustment parameters from the database 50, and the parameter presentation unit 35 presents the adjustment parameters (steps S2 to S4).

When the image quality data does not meet the constraint condition, or when the user does not have particular image quality data and selects a desired image from the image database 60 via the image selection unit 311 (FIG. 3B), only the images meeting the constraint condition received by the constraint condition reception unit 315 are selected and presented, so as to present images from the image database 60 after segmented into predetermined categories, groups, or subgroups (S13). The subsequent steps, i.e., calculating the image feature value for the image selected by the user and determining the suitable adjustment parameters, are the same as those in the first embodiment (steps S2 to S4).

According to the present embodiment, the constraint condition is received in advance, thereby enabling determination of the adjustment parameters in which usable conditions and user's preference are reflected, and this achieves savings in time and effort for the processing such as parameter readjustment performed subsequently. For example, step S5 in FIG. 2 can be skipped.

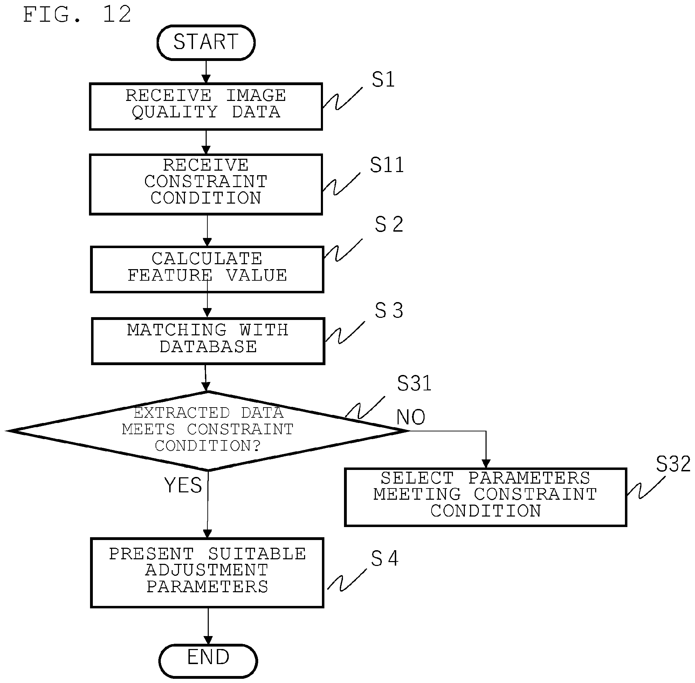

It is to be noted that the modification of the first embodiment can also be applied to the present embodiment. Further, as the modification of the present embodiment, in the above description, the image selection unit 311 selects the images based on the constraint condition, when segmentation of the image database is performed. As shown in FIG. 12, it is further possible that when the data extraction unit 34 extracts specific data (adjustment parameters and images may be included) from the database 50, using the feature value and the DICOM information of the image obtained from the image quality data, the extraction is performed with the constraint condition received by the constraint condition reception unit 315 (S31, S32).

## Claims

1. A medical image processing apparatus (3) comprising
a reception unit (31) configured to select and present multiple candidate images, from multiple images contained in an image database (60), and receive one or more images selected by a user from among the multiple candidate images as image quality data reflecting the user's desire for an image quality of a medical image,
a feature value calculation unit (33) configured to calculate an image feature value with respect to the image quality data, and
a parameter presentation unit (35) configured to use a database (50) associating adjustment parameters related to the image quality with the image feature value, to determine and present the adjustment parameters suitable for the image quality data received by the reception unit (31), wherein the adjustment parameters include imaging conditions or conditions at the time of image generation.

2. The medical image processing apparatus (3) according to claim 1, wherein the image quality data received by the reception unit (31) is DICOM image data including DICOM information.

3. The medical image processing apparatus (3) according to claim 2, wherein the parameter presentation unit (35) is configured to use the image feature value calculated by the feature value calculation unit (33) and the DICOM information to determine the adjustment parameters suitable for the image quality data.

4. The medical image processing apparatus (3) according to any preceding claim, wherein the reception unit (31) is configured to repeat processing steps as the following at least one time: presenting multiple candidate images included in one group among multiple images contained in the image database (60), and after receiving user's selection of a candidate image, presenting another multiple images included in a subordinate group at a level lower than the one group, and then receiving another user's selection of an image.

5. The medical image processing apparatus (3) according to any preceding claim, wherein
the database (50) holds the adjustment parameters and the image feature values in the form of discrete data, and
the database (50) preferably further holds interpolation data that interpolates the discrete data.

6. The medical image processing apparatus (3) according to claim 5, wherein the parameter presentation unit (35) is configured to interpolate the discrete data of the database (50), and use the discrete data and the interpolation data to determine the adjustment parameters suitable for the image quality data.

7. The medical image processing apparatus (3) according to any preceding claim, wherein the reception unit (31) further comprises a constraint condition reception unit (315) configured to receive a condition for the adjustment parameters.

8. The medical image processing apparatus (3) according to claim 7, wherein the reception unit (31) is configured to extract from data contained in the image database (60), image data meeting the constraint condition received by the constraint condition reception unit (315), and present the extracted image data.

9. The medical image processing apparatus (3) according to any preceding claim, wherein
the database (50) contains virtual image-quality images linked with the adjustment parameters, and
the parameter presentation unit (35) is configured to present the virtual image-quality images along with the adjustment parameters.

10. The medical image processing apparatus (3) according to any preceding claim, wherein
the database (50) holds the adjustment parameters and the image feature values created for each of multiple sites, and/or
the database (50) holds the adjustment parameters and the image feature values created using standard phantoms obtained by standardizing respective sites.

11. A diagnostic imaging apparatus (1) comprising
an imaging unit (10),
an image generation unit (20) configured to generate an image using measurement data acquired by the imaging unit (10), and
an image processing unit (30) having the features of the medical image processing apparatus (3) of any preceding claim.

12. The diagnostic imaging apparatus (1) according to claim 11, wherein the diagnostic imaging apparatus (1) is any of an MRI apparatus, a CT apparatus, and an ultrasound imaging apparatus.

13. A medical image processing method comprising
using a reception unit (31) to select and present multiple candidate images, from multiple images contained in an image database (60), and receive (S1) one or more images selected by a user from among the multiple candidate images as image quality data reflecting the user's desire for an image quality of a medical image,
using a feature value calculation unit to calculate (S2) an image feature value with respect to the image quality data, and
using (S3, S4) a database (50) associating adjustment parameters related to the image quality with the image feature value to determine and present the adjustment parameters suitable for the image quality data being received, wherein the adjustment parameters include imaging conditions or conditions at the time of image generation.

14. The medical image processing method according to claim 13, wherein
receiving (S1) the image quality data repeats the following one or more times:
selecting and presenting a group of images from the image database (60),
receiving user's selection regarding the group of images,
selecting and presenting multiple images included in the group of images, or a subordinate group, and
receiving another user's selection regarding the multiple images or the subordinate group, and
an image obtained by a final user's selection is received as the image quality data.

15. The medical image processing method according to claim 13 or 14, further comprising
receiving (S11) a constraint condition regarding the adjustment parameters,
wherein the group of images are selected based on the constraint condition in selecting and presenting the group of images.

## Patentansprüche

1. Verarbeitungsvorrichtung (3) für medizinische Bilder, umfassend
eine Empfangseinheit (31), die so konfiguriert ist, dass sie mehrere Kandidatenbilder aus mehreren Bildern, die in einer Bilddatenbank (60) enthalten sind, auswählt und präsentiert und ein oder mehrere Bilder, die von einem Benutzer aus den mehreren Kandidatenbildern ausgewählt werden, als Bildqualitätsdaten, die den Wunsch seitens von Benutzer nach einer Bildqualität eines medizinischen Bildes wiedergeben, empfängt,
eine Merkmalswert-Berechnungseinheit (33), die so konfiguriert ist, dass sie einen Bildmerkmalswert in Bezug auf die Bildqualitätsdaten berechnet, und
eine Parameterpräsentationseinheit (35), die so konfiguriert ist, dass sie eine Datenbank (50), die Anpassungsparameter, die sich auf die Bildqualität beziehen, dem Bildmerkmalswert zuordnet, verwendet, um die Anpassungsparameter, die für die von der Empfangseinheit (31) empfangenen Bildqualitätsdaten geeignet sind, zu bestimmen und zu präsentieren, wobei die Anpassungsparameter Bildgebungsbedingungen oder Bedingungen zu dem Zeitpunkt von Bilderzeugung umfassen.

2. Verarbeitungsvorrichtung (3) für medizinische Bilder nach Anspruch 1,
wobei die von der Empfangseinheit (31) empfangenen Bildqualitätsdaten DICOM-Bilddaten, die DICOM-Informationen enthalten, sind.

3. Verarbeitungsvorrichtung (3) für medizinische Bilder nach Anspruch 2,
wobei die Parameterpräsentationseinheit (35) so konfiguriert ist, dass sie den von der Merkmalswert-Berechnungseinheit (33) berechneten Bildmerkmalswert und die DICOM-Informationen verwendet, um die für die Bildqualitätsdaten geeigneten Anpassungsparameter zu bestimmen.

4. Verarbeitungsvorrichtung (3) für medizinische Bilder nach einem der vorhergehenden Ansprüche, wobei die Empfangseinheit (31) so konfiguriert ist, dass sie Verarbeitungsschritte als die Folgenden mindestens einmal wiederholt: Präsentieren von mehreren Kandidatenbildern, die in einer Gruppe enthalten sind, unter mehreren Bildern, die in der Bilddatenbank (60) enthalten sind, und, nach Empfangen von Auswahl eines Kandidatenbildes seitens von Benutzer, Präsentieren von weiteren mehreren Bildern, die in einer untergeordneten Gruppe enthalten sind, auf einem Niveau, das niedriger als die eine Gruppe ist, und dann Empfangen einer anderen Auswahl eines Bildes seitens von Benutzer.

5. Verarbeitungsvorrichtung (3) für medizinische Bilder nach einem beliebigen vorhergehenden Anspruch, wobei
die Datenbank (50) die Anpassungsparameter und die Bildmerkmalswerte in der Form von diskreten Daten enthält, und
die Datenbank (50) bevorzugt ferner Interpolationsdaten, die die diskreten Daten interpolieren, enthält.

6. Verarbeitungsvorrichtung (3) für medizinische Bilder nach Anspruch 5,
wobei die Parameterpräsentationseinheit (35) so konfiguriert ist, dass sie die diskreten Daten der Datenbank (50) interpoliert und die diskreten Daten und die Interpolationsdaten verwendet, um die Anpassungsparameter, die für die Bildqualitätsdaten geeignet sind, zu bestimmen.

7. Verarbeitungsvorrichtung (3) für medizinische Bilder nach einem der vorhergehenden Ansprüche, wobei die Empfangseinheit (31) ferner eine Empfangseinheit (315) für Randbedingung umfasst, die so konfiguriert ist, dass sie eine Bedingung für die Anpassungsparameter empfängt.

8. Verarbeitungsvorrichtung (3) für medizinische Bilder nach Anspruch 7,
wobei die Empfangseinheit (31) so konfiguriert ist, dass sie aus Daten, die in der Bilddatenbank (60) enthalten sind, Bilddaten, die die von der Empfangseinheit (315) für Randbedingung empfangene Randbedingung erfüllen, extrahiert, und die extrahierten Bilddaten präsentiert.

9. Verarbeitungsvorrichtung (3) für medizinische Bilder nach einem beliebigen vorhergehenden Anspruch, wobei
die Datenbank (50) virtuelle Bildqualitätsbilder enthält, die mit den Anpassungsparametern verknüpft sind, und
die Parameterpräsentationseinheit (35) so konfiguriert ist, dass sie die virtuellen Bildqualitätsbilder zusammen mit den Anpassungsparametern präsentiert.

10. Verarbeitungsvorrichtung (3) für medizinische Bilder nach einem beliebigen vorhergehenden Anspruch, wobei
die Datenbank (50) die Anpassungsparameter und die Bildmerkmalswerte, die für jede von mehreren Stellen erstellt wurden, enthält, und/oder
die Datenbank (50) die Anpassungsparameter und die Bildmerkmalswerte, die unter Verwendung von Standard-Phantomen, die durch Standardisieren von jeweiligen Stellen erhalten wurden, erstellt wurden, enthält.

11. Diagnostische Bildgebungsvorrichtung (1), umfassend
eine Bildgebungseinheit (10),
eine Bilderzeugungseinheit (20), die so konfiguriert ist, dass sie ein Bild unter Verwendung von Messdaten erzeugt, die von der Bildgebungseinheit (10) erfasst wurden, und
Bildverarbeitungseinheit (30) mit den Merkmalen der Verarbeitungsvorrichtung (3) für medizinische Bilder nach einem der vorhergehenden Ansprüche.

12. Diagnostische Bildgebungsvorrichtung (1) nach Anspruch 11, wobei die diagnostische Bildgebungsvorrichtung (1) eine MRT-Vorrichtung, eine CT-Vorrichtung oder eine Ultraschall-Bildgebungsvorrichtung ist.

13. Verarbeitungsverfahren für medizinische Bilder, umfassend
Verwenden einer Empfangseinheit (31), um mehrere Kandidatenbilder aus mehreren Bildern, die in einer Bilddatenbank (60) enthalten sind, auszuwählen und zu präsentieren, und Empfangen (S1) eines oder mehrerer Bilder, die von einem Benutzer aus den mehreren Kandidatenbildern ausgewählt werden, als Bildqualitätsdaten, die den Wunsch seitens von Benutzer nach einer Bildqualität eines medizinischen Bildes wiedergeben, Verwenden einer Merkmalswert-Berechnungseinheit, um einen Bildmerkmalswert in Bezug auf die Bildqualitätsdaten zu berechnen (S2), und
Verwenden (S3, S4) einer Datenbank (50), die Anpassungsparameter, die sich auf die Bildqualität beziehen, dem Bildmerkmalswert zuordnet, um die Anpassungsparameter, die für die von der Empfangseinheit empfangenen Bildqualitätsdaten geeignet sind, zu bestimmen und zu präsentieren, wobei die Anpassungsparameter Bildgebungsbedingungen oder Bedingungen zu dem Zeitpunkt von Bilderzeugung umfassen.

14. Verarbeitungsverfahren für medizinische Bilder nach Anspruch 13, wobei Empfangen (S1) der Bildqualitätsdaten die Folgenden einmal oder mehrmals wiederholt:
Auswählen und Präsentieren einer Gruppe von Bildern aus der Bilddatenbank (60), Empfangen von Auswahl seitens von Benutzer in Bezug auf die Gruppe von Bildern, Auswählen und Präsentieren von mehreren Bildern, die in der Gruppe von Bildern oder einer untergeordneten Gruppe enthalten sind, und
Empfangen einer anderen Auswahl seitens von Benutzer in Bezug auf die mehreren Bilder oder die untergeordnete Gruppe, und
ein Bild, das durch eine endgültige Auswahl seitens von Benutzer erhalten wird, als die Bildqualitätsdaten empfangen wird.

15. Verarbeitungsverfahren für medizinische Bilder nach Anspruch 13 oder 14, ferner umfassend
Empfangen (S11) einer Randbedingung in Bezug auf die Anpassungsparameter,
wobei die Gruppe von Bildern auf der Grundlage der Randbedingung beim Auswählen und Präsentieren der Gruppe von Bildern ausgewählt wird.

## Revendications

1. Appareil de traitement d'images médicales (3) comprenant
une unité de réception (31) configurée pour sélectionner et présenter plusieurs images candidates, parmi plusieurs images contenues dans une base de données d'images (60), et recevoir une ou plusieurs images sélectionnées par un utilisateur parmi les plusieurs images candidates comme données de qualité d'image reflétant le désir de l'utilisateur pour une qualité d'image d'une image médicale,
une unité de calcul de valeur de caractéristique (33) configurée pour calculer une valeur de caractéristique d'image par rapport aux données de qualité d'image, et
une unité de présentation des paramètres (35) configurée pour utiliser une base de données (50) associant des paramètres d'ajustement liés à la qualité d'image avec la valeur de caractéristique d'image, pour déterminer et présenter les paramètres d'ajustement adaptés pour les données de qualité d'image reçues par l'unité de réception (31), dans lequel les paramètres d'ajustement incluent des conditions d'imagerie ou des conditions au moment de la génération d'images.

2. Appareil de traitement d'images médicales (3) selon la revendication 1,
dans lequel les données de qualité d'image reçues par l'unité de réception (31) sont des données d'image DICOM comprenant des informations DICOM.

3. Appareil de traitement d'images médicales (3) selon la revendication 2,
dans lequel l'unité de présentation des paramètres (35) est configurée pour utiliser la valeur de caractéristique d'image calculée par l'unité de calcul de valeur de caractéristique (33) et les informations DICOM pour déterminer les paramètres d'ajustement adaptés aux données de qualité d'image.

4. Appareil de traitement d'images médicales (3) selon l'une quelconque des revendications précédentes, dans lequel l'unité de réception (31) est configurée pour répéter étapes de traitement suivantes au moins une fois : présenter plusieurs images candidates incluses dans un groupe parmi plusieurs images contenues dans la base de données d'images (60), et après avoir reçu la sélection d'une image candidate de l'utilisateur, présenter d'autres plusieurs images incluses dans un groupe subordonné à un niveau inférieur à celui du groupe, puis recevoir une autre sélection d'image de l'utilisateur.

5. Appareil de traitement d'images médicales (3) selon l'une quelconque des revendications précédentes, dans lequel
la base de données (50) contient les paramètres d'ajustement et les valeurs de caractéristique d'image sous forme de données discrètes, et
La base de données (50) contient en outre de préférence des données d'interpolation qui interpolent les données discrètes.

6. Appareil de traitement d'images médicales (3) selon la revendication 5,
dans lequel l'unité de présentation des paramètres (35) est configurée pour interpoler les données discrètes de la base de données (50), et utiliser les données discrètes et les données d'interpolation pour déterminer les paramètres d'ajustement adaptés pour les données de qualité d'image.

7. Appareil de traitement d'images médicales (3) selon l'une quelconque des revendications précédentes, dans lequel l'unité de réception (31) comprend en outre une unité de réception de condition de contrainte (315) configurée pour recevoir une condition pour les paramètres d'ajustement.

8. Appareil de traitement d'images médicales (3) selon la revendication 7,
dans lequel l'unité de réception (31) est configurée pour extraire des données contenues dans la base de données d'images (60), des données d'image répondant à la condition de contrainte reçue par l'unité de réception de condition de contrainte (315), et présenter les données d'image extraites.

9. Appareil de traitement d'images médicales (3) selon l'une quelconque des revendications précédentes, dans lequel
la base de données (50) contient des images virtuelles de qualité d'image liées aux paramètres d'ajustement, et
l'unité de présentation des paramètres (35) est configurée pour présenter les images virtuelles de qualité d'image ainsi que les paramètres d'ajustement.

10. Appareil de traitement d'images médicales (3) selon l'une quelconque des revendications précédentes, dans lequel
la base de données (50) contient les paramètres d'ajustement et les valeurs de caractéristique d'image créées pour chacun des plusieurs sites, et/ou
la base de données (50) contient les paramètres d'ajustement et les valeurs de caractéristique d'image créées en utilisant fantômes standards obtenus en standardisant des sites respectifs.

11. Appareil d'imagerie diagnostique (1) comprenant
une unité d'imagerie (10),
une unité de génération d'images (20) configurée pour générer une image en utilisant des données de mesure acquises par l'unité d'imagerie (10), et
une unité de traitement d'images (30) ayant les caractéristiques de l'appareil de traitement d'images médicales (3) de l'une quelconque des revendications précédentes.

12. Appareil d'imagerie diagnostique (1) selon la revendication 11, dans lequel l'appareil d'imagerie diagnostique (1) est un d'un appareil IRM, d'un appareil CT, et d'un appareil d'imagerie par ultrasons.

13. Procédé de traitement d'images médicales comprenant
utiliser une unité de réception (31) pour sélectionner et présenter plusieurs images candidates, parmi plusieurs images contenues dans une base de données d'images (60), et recevoir (S1) une ou plusieurs images sélectionnées d'un utilisateur parmi les plusieurs images candidates comme données de qualité d'image reflétant le désir de l'utilisateur pour une qualité d'image d'une image médicale, utiliser une unité de calcul de valeur de caractéristique pour calculer (S2) une valeur de caractéristique d'image par rapport aux données de qualité d'image, et
utiliser (S3, S4) une base de données (50) associant des paramètres d'ajustement liés à la qualité d'image avec la valeur de caractéristique d'image pour déterminer et présenter les paramètres d'ajustement adaptés pour les données de qualité d'image reçues, dans lequel les paramètres d'ajustement incluent des conditions d'imagerie ou des conditions au moment de la génération d'images.

14. Procédé de traitement d'images médicales selon la revendication 13, dans lequel recevoir (S1) des données de qualité d'image répète une ou plusieurs fois ce qui suit :
sélectionner et présenter un groupe d'images à partir de la base de données d'images (60),
recevoir la sélection par l'utilisateur concernant le groupe d'images,
sélectionner et présenter plusieurs images incluses dans le groupe d'images ou dans un groupe subordonné, et
recevoir une autre sélection de la utilisateur concernant les plusieurs images ou le groupe subordonné, et
une image obtenue par une sélection finale de la utilisateur est reçue comme les données de qualité d'image.

15. Procédé de traitement d'images médicales selon la revendication 13 ou la revendication 14, comprenant en outre
recevoir (S11) une condition de contrainte concernant les paramètres d'ajustement,
dans lequel le groupe d'images est sélectionné sur la base de la condition de contrainte lors de la sélection et de la présentation du groupe d'images.
